# EUROPÄISCHE PATENTSCHRIFT

(11) **EP 0 515 721 B1**
(45) Veröffentlichungstag und Bekanntmachung des Hinweises auf die Patenterteilung: **12.01.1994**
(21) Anmeldenummer: 91108910.0
(22) Anmeldetag: 31.05.1991
(51) Int. Cl.: A23L 1/015, A23L 2/34, A23L 1/212

(54) **Verfahren zur Herstellung pflanzlicher Lebensmittel mit geringem Nitratgehalt**
Method for producing vegetable foodstuff with low nitrate content
Procédé de préparation d'aliments végétaux à faible teneur en nitrate

(43) Veröffentlichungstag der Anmeldung: 02.12.1992
(73) Patentinhaber: Eckes Aktiengesellschaft, D-55268 Nieder-Olm (DE)
(72) Erfinder: Wiesenberger, Alfred, W-6200 Wiesbaden-Sonnenberg (DE); Kolb, Erich Dr. Dipl.-Ing., W-6501 Nieder-Olm (DE); Haug, Martin Dr., W-6509 Schornsheim (DE)
(74) Vertreter: Gudel, Diether

(56) Entgegenhaltungen:
- EP-A- 0 244 663
- DE-A- 1 801 295

## Beschreibung

Die Erfindung betrifft ein Verfahren zur Herstellung pflanzlicher Lebensmittel mit geringem Nitratgehalt, insbesondere aus Gemüse, wobei weitgehend von Fremdkeimen befreites pflanzliches Material des Lebensmittels mit geeigneten denitrifizierenden Mikroorganismen versetzt wird, die den Nitratgehalt des pflanzlichen Materials verringern, ohne dabei dessen Charakter merklich zu verändern.

Ein derartiges Verfahren ist in der EP-244 663-A3 beschrieben. Man macht sich dort die denitrifizierenden Mikroorganismen oder Bakterien zunutze, um den Nitratgehalt auf ernährungsphysiologisch unbedenkliche Werte abzusenken. Die verwendeten Bakterien sind nämlich in der Lage, organische Inhaltsstoffe pflanzlicher Lebensmittel für ihr anaerobes Wachstum zu nutzen, wobei gleichzeitig der Nitratgehalt des pflanzlichen Lebensmittels verringert wird. Dabei wird aber nur ein geringer Anteil der reichlich vorhandenen organischen Inhaltsstoffe für das Wachstum der Bakterien benötigt, so daß der Charakter des Lebensmittels praktisch nicht verändert wird, zumal der Anteil der Zellmasse der Bakterien gering ist. Der Charakter des pflanzlichen Materials bzw. Lebensmittels bezeichnet hierbei insbesondere die Zusammensetzung, den Geschmack und das Aussehen des Lebensmittels, so daß das Endprodukt nach der Behandlung vom Ausgangsprodukt für den Verbraucher nicht mehr unterschieden werden kann; es zeichnet sich lediglich durch einen ungefährlichen Nitratgehalt aus.

Versuche der Anmelderin haben aber ergeben, daß es nach dem dort beschriebenen Verfahren während des Nitratabbaus zu intermediären Nitritanhäufungen kommt. Diese intermediären Nitritanhäufungen treten insbesondere bei den natürlicherweise vorhandenen pH-Werten (3,0 - 6,0) von pflanzlichem Material auf. Nach den Erkenntnissen der Anmelderin ist diese Nitritanhäufung bei den erwähnten pH-Werten bei unterschiedlichen DSM Stämmen des Paracoccus zu beobachten.

Eine Nitritanhäufung kann aber zur Bildung von Nitrosaminen aus im Lebensmittel (Saft) anwesenden Inhaltsstoffen führen. In der erwähnten Offenlegungsschrift wird zwar berichtet, daß im behandelten Produkt Nitrosamine nicht nachgewiesen wurden, dies schließt aber nicht aus, daß bisher nicht untersuchte Nitrosamine entstanden sind. Auch variieren Rohstoff und Fermentationsbedingungen und auch die Höhe und die Dauer der Nitritanhäufung bei einem solchen Verfahren, weshalb eine Nitrosaminbildung nicht sicher auszuschließen ist. Dies zeigen Versuche der Anmelderin, nach welchen es bei Zusatz einer leicht nitrosierbaren Verbindung während der Denitrifizierung zu einer Nitrosaminbildung kommen kann, wenn keine Gegenmaßnahmen getroffen werden. Die Bildung von Nitrosaminen ist aber gefährlich, weil einige bekanntlich im Verdacht stehen, kanzerogen zu wirken.

Die deutsche Patentschrift 18 01 295 lehrt, zur Verhinderung der Nitritbildung Spinat mit einer ungiftigen Säure zu versetzen, z.B. mit Ascorbinsäure oder Zitronensäure, weil durch den Säurezusatz das Wachstum der nitritbildenden Spontanflora unterdrückt wird. Der Nitratgehalt des Spinats bleibt hierbei aber unverändert. Auf eine mögliche Vermeidung der Nitrosaminbildung wird dort nicht Bezug genommen.

Die Erfindung vermeidet diese Nachteile. Ihr liegt die Aufgabe zugrunde, ein Verfahren mit den Merkmalen des Oberbegriffs des Hauptanspruchs vorzuschlagen, bei dem die Bildung toxischer Stoffwechselprodukte bei der Nitratentfernung oder Nitratverringerung vermieden wird.

Zur Lösung dieser Aufgabe ist die Erfindung dadurch gekennzeichnet, daß zunächst eine Vorkultur aus dem pflanzlichen Material und den Mikroorganismen hergestellt wird, die einen pH-Wert zwischen 6,3 und 8 hat und die dann zum Nitratabbau dem noch nicht mit den Mikroorganismen behandelten pflanzlichen Material in einer Menge zugegeben wird, daß das Gemisch einen pH-Wert zwischen 6 und 7,5 erhält, worauf der Nitratabbau erfolgt, wobei nach der Zugabe der Vorkultur oder auch bereits in der Vorkultur zu dem pflanzlichen Material dem Gemisch Ascorbinsäure, Ester der Ascorbinsäure und/oder deren Salze in Mengen von 10 mg/l bis 1000 mg/l zugegeben werden.

Durch diese Maßnahmen wird der pH-Wert entsprechend angehoben, wodurch die Nitritbildung verringert wird. Es brauchen somit keine Puffer oder Laugen zugesetzt zu werden, wodurch an und für sich auch der pH-Wert entsprechend angehoben werden könnte. Der Zusatz von Puffern oder Laugen verbietet sich aber bei den meisten pflanzlichen Lebensmitteln, beispielsweise auch bei Gemüsesäften, aufgrund rechtlicher Vorschriften und sensorischen Veränderungen.

Mit der erfindungsgemäßen Verfahrensführung wird erreicht, daß in der Voranzucht der denitrifizierenden Mikroorganismen in pflanzlichem Material rasch pH-Werte über 6 erreicht werden. Diese Voranzucht setzt man in noch nicht behandeltes pflanzliches Material ein, so daß von vornherein ein pH-Wert über 6 erreicht wird, wodurch die Denitrifizierung beschleunigt wird und die Höhe und Dauer der Nitritakkumulation verringert wird.

Es können dem Gemisch auch Vitamine E in Form von Tocopherolen und/oder deren Acetate in Mengen von 2 mg/l bis 500 mg/l zugegeben werden.

Erst bei der vorliegenden Erfindung wird berücksichtigt, daß bei der Verfahrensführung nach der erwähnten EP-A3 mit der Möglichkeit gerechnet werden muß, daß toxische Stoffwechselprodukte auftreten können. Es gehört auch zur erfindungsgemäßen Erkenntnis, daß die der Lösung der Erfindungsaufgabe besonders förderlichen Zusätze nach den Patentansprüchen 2 und 3 während des Nitratabbaus einsetzbar sind, ohne daß der Prozeß der Nitratentfernung hierbei negativ beeinflußt wird. Eine eventuelle Nitrosaminbildung wird dadurch effektiv unterdrückt. Da die Nitratentfernung mit einem pH-Wert-Anstieg verbunden ist, ist es auch möglich, den Ascorbinsäurezusatz kontinuierlich ab beginnender Nitritanhäufung vorzunehmen.

### Anwendungsbeispiele:

### Beispiel 1:

a.) Herstellung der Vorkultur
   1 l steriler Rote-Bete-Saft wurde mit 20 g feuchter Biomasse einer Reinkultur von P. denitrificans DSM 1403 versetzt. Die Flüssigkeit wurde unter sterilen Bedingungen für 5 h bei 30°C unter Stickstoffatmosphäre gehalten. Der Ausgangsnitratgehalt der Flüssigkeit von 1950 mg/l nahm während dieser Zeit auf 50 mg/l ab, der pH-Wert stieg von 5,7 auf 7,6.
b.) Nitratabbau
   Die unter a.) erhaltene Flüssigkeit wurde unter sterilen Bedingungen zu 4 l sterilen Rote-Bete-Saftes gegeben. Der ursprüngliche pH-Wert des Saftes stieg dadurch von 5,7 auf 6,6. Unter sterilen Bedingungen wurde der Saft 10 h bei 30°C gehalten. Der pH-Wert steigt während dieser Zeit auf 7,6. Der Nitratgehalt nahm von 1950 auf unter 25 mg/l ab. Der intermediäre Nitritgehalt lag für 5 h zwischen 25-50 mg/l; im Endprodukt war kein Nitrit mehr nachweisbar.

### Beispiel 2:

a.) Herstellung der Vorkultur
   20 l steriler Sellerie-Saft wurde mit 200 g feuchter Biomasse einer Reinkultur von P. denitrificans DSM 65 versetzt. Die Flüssigkeit wurde unter sterilen Bedingungen für 2 h bei 25°C unter Luftabschluß gehalten. Der Ausgangsnitratgehalt der Flüssigkeit von 550 mg/l nahm während dieser Zeit auf 25 mg/l ab, der pH-Wert stieg von 5,3 auf 6,3.
b.) Nitratabbau
   Die unter a.) erhaltene Flüssigkeit wurde unter sterilen Bedingungen zu 60 l sterilen Sellerie-Saftes gegeben. Der ursprüngliche pH-Wert des Saftes stieg dadurch von 5,3 auf 6,0. Unter sterilen Bedingungen wurde der Saft 5 h bei 25°C gehalten. Bei Erreichen von pH 6,5 wurde zur Konstanthaltung des pH-Wertes, über eine sterile Dosiervorrichtung Zitronensaftkonzentrat zugegeben. Der Nitratgehalt nahm von 550 auf unter 25 mg/l ab. Der intermediäre Nitritgehalt lag für 2,5 h zwischen 50 - 75 mg/l; im Endprodukt war kein Nitrit mehr nachweisbar.

### Beispiel 3:

a.) Herstellung der Vorkultur
   10 l Rote-Bete-Saft wurde mit 100 mg Ascorbinsäure/l versetzt und sterilisiert. Der Saft wurde mit 150 g feuchter Biomasse einer Reinkultur P. denitrificans DSM 65 versetzt. Die Flüssigkeit wurde unter sterilen Bedingungen für 5 h bei 32°C unter Luftabschluß gehalten. Der Ausgangsnitratgehalt der Flüssigkeit von 1050 mg/l nahm während dieser Zeit auf 25 mg/l ab, der pH-Wert stieg von 5,5 auf 7,0.
b.) Nitratabbau
   Die unter a.) erhaltene Flüssigkeit wurde unter sterilen Bedingungen zu 50 l sterilen Rote-Bete-Saft, dem 100 mg Ascorbinsäure/l zugesetzt worden waren, gegeben. Der ursprüngliche pH-Wert des Saftes stieg dadurch von 5,5 auf 6,4. Unter sterilen Bedingungen wurde der Saft 8 h bei 32°C gehalten. Bei Erreichen von pH 6,5 wurde zur Konstanthaltung des pH-Wertes über eine sterile Dosiervorrichtung Zitronensaftkonzentrat zugegeben. Der Nitratgehalt nahm von 1.050 auf unter 25 mg/l ab. Der intermediäre Nitritgehalt lag für 2,5 h zwischen 25-50 mg/l; im Endprodukt war kein Nitrit mehr nachweisbar. Eine während des Prozesses gezogene Probe, die noch 50 mg Nitrit/l enthielt, wurde mit einer leicht nitrosierbaren Verbindung versetzt, wobei innerhalb von 2,5 h keine Bildung des entsprechenden Nitrosamins nachgewiesen werden konnte.(Nachweisgrenze < 0,5 ppb.)
c.) Ansäuerung des Saftes
   Der unter b.) erhaltene Saft wurde mit 1500 mg Ascorbinsäure/l versetzt, wobei ein pH-Wert von 4,7 erreicht wurde.

### Beispiel 4:

a.) Herstellung der Vorkultur
   10 l Karottensaft wurden mit 100 mg Ascorbinsäure/l und 15 mga Tocopherol/l versetzt und sterilisiert. Der Saft wurde mit 100 g feuchter Biomasse einer Reinkultur von P. denitrificans DSM 1403 versetzt. Die Flüssigkeit wurde unter sterilen Bedingungen für 5 h bei 32°C unter Luftabschluß gehalten. Der Ausgangsnitratgehalt der Flüssigkeit von 420 mg/l nahm während dieser Zeit auf 25 mg/l ab, der pH-Wert stieg von 5,4 auf 6,9.
b.) Nitratabbau
   Die unter a.) erhaltene Flüssigkeit wurde unter sterilen Bedingungen zu 50 l sterilen Karottensaft, dem 100 mg Ascorbinsäure/l und 15 mga Tocopherol/l zugesetzt worden waren, gegeben. Der ursprüngliche ph-Wert des Saftes stieg dadurch von 5,4 auf 6,4. Unter sterilen Bedingungen wird der Saft 8 h bei 32°C gehalten. Bei Erreichen von pH 6,5 wurde zur Konstanthaltung des pH-Wertes, über eine sterile Dosiervorrichtung Zitronensaftkonzentrat zugegeben. Der Nitratgehalt nahm von 550 auf unter 25 mg/l ab. Der intermediäre Nitritgehalt lag für 2,5 h zwischen 10 - 25 mg/l; im Endprodukt war kein Nitrit mehr nachweisbar. Eine während des Prozesses gezogene Probe, die noch 25 mg Nitrit/l enthielt, wurde mit einer leicht nitrosierbaren Verbindung versetzt, wobei innerhalb von 8 h keine Bildung des entsprechenden Nitrosamins nachgewiesen werden konnte. (Nachweisgrenze < 0,5 ppb).
c.) Ansäurung des Saftes
   Der unter b.) erhaltene Saft wurde mit 1500 mg Ascorbinsäure/l versetzt, wobei ein pH-Wert von 4,9 erreicht wurde.

### Beispiel 5:

Bei Herstellung der Voranzucht und Nitratabbau wurde wie unter Beispiel 2 - 4 verfahren. Die jeweiligen Zusätze Ascorbinsäure, Vitamin E, Genußsäuren und Zitronensäure wurden in einen Teil des entsprechenden Gemüsesaftes eingerührt und die Mischung sterilisiert. Die jeweilige Mischung wurde während des Nitratabbaus zur Konstanthaltung des pH-Wertes und Zugabe der Zusätze steril eindosiert.

### Beispiel 6:

Bei Herstellung der Voranzucht und Nitratabbau wurde wie unter Beispiel 1 - 4 verfahren. Die Ascorbinsäure wurde in Form von sterilem Acerolamark, welches 1 g Ascorbinsäure/l enthält, zugegeben. Der Zusatz kann batchweise oder kontinuierlich erfolgen.

## Patentansprüche

1. Verfahren zur Herstellung pflanzlicher Lebensmittel mit geringem Nitratgehalt, insbesondere aus Gemüse, wobei weitgehend von Fremdkeimen befreites pflanzliches Material des Lebensmittels mit geeigneten denitrifizierenden Mikroorganismen versetzt wird, die den Nitratgehalt des pflanzlichen Materials verringern, ohne dabei dessen Charakter merklich zu ändern,
**dadurch gekennzeichnet**,
daß zunächst eine Vorkultur aus dem pflanzlichen Material und den Mirkoorganismen hergestellt wird, die einen pH-Wert zwischen 6,3 und 8 hat und die dann zum Nitratabbau dem noch nicht mit den Mikroorganismen behandelten pflanzlichen Material in einer Menge zugegeben wird, daß das Gemisch einen pH-Wert zwischen 6 und 7,5 erhält, worauf der Nitratabbau erfolgt, wobei nach der Zugabe der Vorkultur zu dem pflanzlichen Material dem Gemisch Ascorbinsäure, Ester der Ascorbinsäure und/oder deren Salze in Mengen von 10 mg/l bis 1.000 mg/l zugegeben werden.

2. Verfahren nach Anspruch 1,
**dadurch gekennzeichnet**,
daß dem Gemisch Vitamin E in Form von Tocopherolen (a, β, g, d -Tocopherol) und/oder deren Acetate in Mengen von 2 mg/l bis 500 mg/l zugegeben werden.

3. Verfahren nach Anspruch 1 oder 2,
**dadurch gekennzeichnet**,
daß die Zusätze während des Nitratabbaus kontinuierlich zugegeben werden.

4. Verfahren nach einem der Ansprüche 1 bis 3,
**dadurch gekennzeichnet**,
daß als denitrifizierende Mikroorganismen Bakterien aus der Gattung Pseudomonas stutzeri oder denitrificans, Paracoccus denitrificans oder Bacillus licheniformis eingesetzt werden.

5. Verfahren nach einem der Ansprüche 1 bis 4,
**dadurch gekennzeichnet**,
daß als denitrifizierender Mikroorganismus Paracoccus denitrificans DSM 65 oder DSM 1403 eingesetzt wird.

6. Verfahren nach einem der Ansprüche 1 bis 5,
**dadurch gekennzeichnet**,
daß nach der Nitratentfernung der pH-Wert des pflanzlichen Materials durch Genußsäuren, Ascorbinsäure und/oder Fruchtsaftkonzentrate auf Werte zwischen 6 und 3,5 eingestellt wird.

7. Verfahren nach einem der Ansprüche 1 bis 6,
**dadurch gekennzeichnet**,
daß zur Konstanthaltung des ph-Wertes während des Nitratabbaus zusätzlich Säure in Form von Fruchtsaft, Fruchtsaftkonzentraten und/oder Genußsäuren oder Ascorbinsäure zugegeben wird.

8. Verfahren nach einem der Ansprüche 1 bis 7,
**dadurch gekennzeichnet**,
daß die Vorkultur dem noch nicht behandelten pflanzlichen Material im Verhältnis 1 : 10 bis 1 : 1 Gewichtsanteilen zugeben wird.

9. Verfahren nach einem der Ansprüche 1 bis 8,
**dadurch gekennzeichnet**,
daß die Zusätze der Patentansprüche 1 und/oder 2 bereits zur Vorkultur zugegeben werden

## Claims

1. A method for producing vegetable foodstuff with a low nitrate content, more particularly from vegetables, vegetable foodstuff material extensively freed from foreign germs being mixed with suitable denitrifying microorganisms, which reduce the nitrate content of the vegetable material without thereby noticeably altering the character of the said vegetable material, characterised in that a pre-culture is firstly produced from the vegetable material and the microorganisms, which has a pH value between 6.3 and 8 and which is then added for nitrate reduction to the vegetable material as yet untreated with the microorganisms in such a quantity that the fixture reaches a pH value of between 6 and 7.5, whereupon the nitrate reduction occurs, and following the addition of the pre-culture to the vegetable material ascorbic acid, esters of ascorbic acid and/or the salts thereof are added to the mixture in quantities of 10 mg/l to 1,000 mg/l.

2. A method according to claim 1, characterised in that vitamin E in the form of tocopherols (a, β, g, d - tocopherol) and/or the acetates thereof is added to the mixture in quantities of 2 mg/l to 500 mg/l.

3. A method according to claim 1 or 2, characterised in that the additions are added continuously during the nitrate reduction.

4. A method according to one of claims 1 to 3, characterised in that bacteria of the type pseudomonas stutzeri or denitrificans, paracoccus denitrificans or bacillus licheniformis are used as denitrifying microorganisms.

5. A method according to one of claims 1 to 4, characterised in that paracoccus denitrificans DSM 65 or DSM 1403 is used as a denitrifying microorganism.

6. A method according to one of claims 1 to 5, character-ised in that, after the nitrate removal, the pH value of the vegetable material is adjusted to values between 6 and 3.5 by edible acids, ascorbic acid and/or fruit juice concentrates.

7. A method according to one of claims 1 to 6, characterised in that additional acids in the form of fruit juice, fruit juice concentrates and/or edible acids or ascorbic acids are added in order to keep the pH value constant during the nitrate reduction.

8. A method according to one of claims 1 to 7, characterised in that the pre-culture is added to the as yet untreated vegetable material in a ratio of 1 : 10 to 1 : 1 parts by weight.

9. A method according to one of claims 1 to 8, characterised in that the additions of claims 1 and/or 2 are already added to the pre-culture.

## Revendications

1. Procédé de préparation d'aliments végétaux ayant une faible teneur en nitrates, en particulier àpartir de légumes, dans lequel la matière végétale de l'aliment largement exempte de germes étrangers est mise en réaction avec des micro-organismes de dénitrification appropriés, qui réduisent la teneur en nitrates de la matière végétale, sans modifier de manière notable son caractère, caractérisé en ce que l'on prépare tout d'abord une préculture à partir de la matière végétale et des micro-organismes, laquelle préculture a une valeur du pH entre 6,3 et 8, et on l'ajoute ensuite, pour dégrader les nitrates, à la matière végétale qui n'a pas encore été traitée par les micro-organismes en quantité telle que le mélange reçoit une valeur du pH comprise entre 6 et 7,5, après quoi il se produit un dénitrification au cours de laquelle, après l'addition de la préculture à la matière végétale, on ajoute au mélange de l'acide ascorbique, des esters d'acide ascorbique et/ou leurs sels en quantité de 10 à 1.000 mg par litre.

2. Procédé selon la revendication 1, caractérisé en ce qu'on ajoute au mélange de la vitamine E sous la forme de tocophérols (a, β, g, d -tocophérols) et/ou leurs acétates en quantité de 2 à 500 mg par litre.

3. Procédé selon la revendication 1 ou 2, caractérisé en ce que les additions sont faites en continu au cours de la dénitrification.

4. Procédé selon l'une des revendications 1 à 3, caractérisé en ce que l'on utilise comme micro-organismes de dénitrification des bactéries du genre Pseudomonas stutzeri ou denitrificans, Paracoccus denitrificans ou Bacillus licheniformis.

5. Procédé selon l'une des revendications 1 à 4, caractérisé en ce qu'on utilise comme micro-organismes dénitrificateurs le Paracoccus denitrificans DSM 65 ou DMS 1403.

6. Procédé selon l'une des revendications 1 à 5, caractérisé en ce que, après la dénitrification, on règle la valeur du pH de la matière végétale à des valeurs comprises entre 6 et 3,5 par addition d'acides alimentaires, d'acide ascorbique et/ou de concentrés de jus de fruit.

7. Procédé selon l'une des revendications 1 à 6, caractérisé en ce que, pour conserver constante la valeur du pH au cours de la dénitrification, on utilise en outre des acides sous forme de jus de fruit, de concentrés de jus de fruit et/ou d'acides alimentaires ou d'acide ascorbique.

8. Procédé selon l'une des revendications 1 à 7, caractérisé en ce que la préculture est ajoutée à la matière végétale non encore traitée à raison de 1:10 à 1:1 parties en poids.

9. Procédé l'une des revendications 1 à 8, caractérisé en ce que les additions de la revendication 1 et/ou 2 sont déjà faites dans la préculture.
